# FASCICULE DE BREVET EUROPEEN

(11) **EP 0 962 224 B1**
(45) Date de publication et mention de la délivrance du brevet: **03.11.2004**
(21) Numéro de dépôt: 99401136.9
(22) Date de dépôt: 07.05.1999
(51) Int. Cl.: A61K 7/48

(54) **Azurants optiques comme agents blanchissants**
Optische Aufheller zur hautbleichung
Optical azurants as skin bleaching agent

(30) Priorité: 14.05.1998 FR 9806109
(43) Date de publication de la demande: 08.12.1999
(73) Titulaire: L'OREAL, 75008 Paris (FR)
(72) Inventeur: Chevalier, Véronique, 94440 Villecresnes (FR); Quest, Mélanie, 75013 Paris (FR)
(74) Mandataire: Renard, Emmanuelle

(56) Documents cités:
- DATABASE WPI Section Ch, Week 9624 Derwent Publications Ltd., London, GB; Class A96, AN 96-235933 XP002098943 & JP 08 092053 A (MIKIMOTO SEIYAKU KK), 9 avril 1996 (1996-04-09)
- DATABASE WPI Section Ch, Week 9441 Derwent Publications Ltd., London, GB; Class B02, AN 94-329890 XP002098944 & JP 06 256150 A (MARUZEN SEIYAKU KK), 13 septembre 1994 (1994-09-13)
- DATABASE WPI Section Ch, Week 9125 Derwent Publications Ltd., London, GB; Class D21, AN 91-181427 XP002098945 & JP 03 109343 A (MARUZEN KASEI CO LTD), 9 mai 1991 (1991-05-09)
- DATABASE WPI Section Ch, Week 8912 Derwent Publications Ltd., London, GB; Class D21, AN 89-088653 XP002098946 & JP 01 038009 A (POLA KASEI KOGYO KK), 8 février 1989 (1989-02-08)
- PATENT ABSTRACTS OF JAPAN vol. 017, no. 561 (C-1119), 8 octobre 1993 (1993-10-08) & JP 05 163115 A (KANEBO LTD), 29 juin 1993 (1993-06-29)
- PATENT ABSTRACTS OF JAPAN vol. 017, no. 674 (C-1140), 10 décembre 1993 (1993-12-10) & JP 05 221845 A (HISAMITSU PHARMACEUT CO INC), 31 août 1993 (1993-08-31)
- PATENT ABSTRACTS OF JAPAN vol. 096, no. 009, 30 septembre 1996 (1996-09-30) & JP 08 127525 A (KANEBO LTD), 21 mai 1996 (1996-05-21)
- DATABASE WPI Section Ch, Week 9519 Derwent Publications Ltd., London, GB; Class B03, AN 95-144683 XP002098947 & JP 07 069859 A (SHISEIDO CO LTD), 14 mars 1995 (1995-03-14)
- CHEMICAL ABSTRACTS, vol. 83, no. 19, 10 novembre 1975 (1975-11-10) Columbus, Ohio, US; abstract no. 158891, XP002116626 & FORBES P. D. ET AL.: FOOD COSMETIC TOXICOL., vol. 13, no. 3, 1975, pages 335-7, USA

## Description

L'invention a pour objet l'utilisation de composés de la famille des azurants optiques dans une composition cosmétique ou pour la préparation d'une composition dermatologique, à application topique, cette composition ayant la propriété de conférer au teint une plus grande uniformité, une plus grande homogénéité, une plus grande transparence, un aspect de porcelaine, une plus grande blancheur. De telles compositions sont également dotées de propriétés anti-cernes.

L'invention a également pour objet l'association de composés de la famille des azurants optiques avec certains composés cosmétiques tels que des agents de blanchiment de la peau, des filtres, des anti-rides et des agents hydratants, ces associations ayant des propriétés particulièrement remarquables dans les axes définis ci-dessus.

Enfin, l'invention a pour objet un procédé de blanchiment immédiat de la peau, ce procédé consistant à appliquer sur la peau une composition cosmétique comprenant au moins un composé de la famille des azurants optiques.

Il est fréquent que les personnes ayant une peau colorée, voire foncée, désirent s'éclaircir la peau et utilisent dans ce but des compositions cosmétiques ou dermatologiques contenant des agents de blanchiment.

Les substances les plus utilisées comme agent de blanchiment sont l'hydroquinone et ses dérivés, l'acide kojique et ses dérivés, l'acide azélaïque, l'arbutine et ses dérivés, seuls ou en association avec d'autres actifs.

De telles compositions comprenant des agents de blanchiment sont également utilisées par les personnes dont la peau présente des dyschromies, ces dyschromies pouvant être d'origines diverses : âge (tâches de vieillesse), exposition aux UV, masque de grossesse, pathologie de la peau...

Toufefois, ces agents ne sont pas dépourvus d'inconvénients. En particulier, il est nécessaire de les utiliser de façon prolongée et en des quantités élevées pour observer un effet de blanchiment de la peau. On n'observe pas un effet immédiat à l'application de compositions les comprenant.

En outre, l'hydroquinone et ses dérivés sont utilisés en quantité efficace pour voir apparaître un effet blanchissant. En particulier, l'hydroquinone est connue pour sa cytotoxicité vis-à-vis du mélanocyte.

Par ailleurs, l'acide kojique et ses dérivés présentent l'inconvénient d'être coûteux et de ne pouvoir, pour cette raison, être utilisés en quantité importante dans des produits à large diffusion commerciale.

Il est connu d'utiliser des compositions cosmétiques susceptibles d'uniformiser le teint, et éventuellement de conférer un aspect blanc immédiat, ces compositions étant constituées de poudres dispersées dans un liant. Les poudres contiennent généralement des pigments blancs ou colorés suivant l'effet souhaité, et des charges sous forme lamellaire, ou encore la silice sous forme de plaquettes. L'uniformisation du teint est obtenue essentiellement grâce au pouvoir couvrant apporté par les charges sous forme lamellaire.

L'inconvénient de telles compositions est que l'estompage des défauts de la peau est apporté par le pouvoir couvrant des compositions. La peau ainsi maquillée perd son aspect naturel du fait du manque de transparence de ces compositions.

Si l'utilisation de charges lamellaires a pu être considéré comme un moyen d'augmenter la transparence de ces compositions (voir par exemple US 4 899 163), elles présentent l'inconvénient d'agir par réflexion de la lumière et confèrent à la peau un aspect brillant peu naturel.

Il subsiste donc le besoin de compositions cosmétiques et/ou dermatologiques permettant d'obtenir un teint blanc, uniforme, homogène, d'aspect naturel, ces compositions présentant une transparence satisfaisante après application sur la peau.

La demande de brevet française publiée sous le numéro FR-2 741 261 décrit des compositions cosmétiques comprenant un agent fluorescent d'avivage également connus comme azurants optiques. Ces agents présentent l'avantage d'intensifier l'éclat et d'aviver les teintes des compositions cosmétiques les comprenant à l'application sur la peau ou les cheveux. L'effet de ces agents est observé sur une support coloré (les cheveux) ou dans une composition colorée (maquillage tel que mascara, vemis-à-ongles, rouge-à-lèvres). Toutefois, l'effet des azurants optiques sur la peau elle-même tel que décrit ci-dessus n'est ni mentionné, ni suggéré dans ce document.

L'invention a pour objet l'utilisation d'au moins un composé appartenant à la famille des azurants optiques, dans une composition ou pour la préparation d'une composition destinée au blanchiment de la peau, en particulier au blanchiment immédiat de la peau. Une telle composition confère au teint une plus grande uniformité, une plus grande homogénéité, une plus grande transparence, un aspect de porcelaine.

Une telle utilisation est particulièrement efficace sur les peaux asiatiques.

Plus particulièrement, cette composition est une composition cosmétique. L'utilisation en dermatologie peut être également envisagée.

De préférence, les compositions selon l'invention sont des compositions pour le soin de la peau et ne sont pas des compositions de maquillage ou des compositions destinées à être appliquées sur les cheveux.

L'invention a également pour objet l'utilisation d'au moins un composé appartenant à la famille des azurants optiques dans une composition ou pour la préparation d'une composition efficace sur les peaux caucasiennes.

Les azurants optiques sont des composés bien connus de l'homme du métier. De tels composés sont décrits dans "Fluorescent Whitening Agent, Encyclopedia of Chemical Technology, Kirk-Othmer", vol 11, p. 227-241, 4^{ème} édition, 1994, Wiley.

On peut plus particulièrement les définir comme des composés qui absorbent essentiellement dans l'UVA entre 300 et 390 nm et réémettent essentiellement entre 400 et 525 nm.

Parmi les azurants optiques, on s'est intéressé particulièrement aux dérivés du stilbène, aux dérivés coumariniques, aux dérivés oxazole et benzoxazole, aux dérivés imidazole.

De tels composés sont facilement disponibles commercialement. On peut citer par exemple le Tinopal SOP ® et l'Uvitex OB ® commercialisés par la société CIBA GEIGY.

Les azurants optiques préférentiellement utilisés selon l'invention sont le 4,4'-bis[(4,6-diamilino-1,3,5-triazin-2-yl)amino]stilbène-2,2'-disulfonate de sodium, le 2,5 thiophène di-yl bis(5 ter-butyl-1,3 benzoxazole).

L'invention a également pour objet l'utilisation d'au moins un composé de la famille des azurants optiques dans une composition ou pour la préparation d'une composition comme agent de blanchiment de la peau, en particulier comme agent de blanchiment immédiat, et/ou comme agent destiné à conférer à la peau au moins l'une des propriétés choisies parmi : uniformité, homogénéité, transparence et aspect de porcelaine.

L'invention a également pour objet l'utilisation d'au moins un composé de la famille des azurants optiques dans une composition ou pour la préparation d'une composition comme agent anti-cernes.

Ainsi, les cernes constatées par la présence d'une surface plus foncée, voir grise ou noire, autour des yeux et/ou par la présence de poches sous les yeux et/ou par un aspect flétri du contour des yeux peuvent diminuer, voire disparaître, grâce à l'application sur celles-ci de la composition selon l'invention.

Les azurants optiques sont utilisés selon la présente invention de préférence en quantité allant de 0,1 à 15 % en poids, et encore plus préférentiellement de 0,5 à 10 % en poids par rapport au poids total de la composition.

De façon avantageuse, les azurants optiques sont associés à d'autres agents cosmétiques avec lesquels ils ont une action renforcée ou complémentaire.

Ainsi, l'invention a pour objet une composition, plus particulièrement cosmétique, comprenant au moins un composé de la famille des azurants optiques et au moins un autre agent de blanchiment et/ou dépigmentant classiquement utilisé en cosmétique.

Parmi ces agents de blanchiment et/ou dépigmentant classiquement utilisés en cosmétique, on préfère utiliser les composés choisis parmi : l'hydroquinone et ses dérivés tels que le monométhyléther, l'hydroquinone, le monoéthyléther d'hydroquinone, l'acide kojique et ses dérivés, l'acide lactique et ses sels, l'acide ascorbique et ses dérivés, l'acide azélaïque, l'arbutine et ses dérivés, l'acide L-2-oxothiazolidine 4-carboxylique (tel que décrit dans le brevet EP 0 780 120), le paracétamol, tel que décrit dans la demande de brevet français déposée par la Demanderesse le 6.12.96 sous le n° 9615046, et des dérivés d'aminophénol à fonction carbamate, urée ou sulfonamide de formule (I), tel que décrit dans la demande de brevet français déposée par la Demanderesse le 27.08.97 sous le n° 9710710. Ces dérivés d'aminophénol présentent la formule (I) suivante : 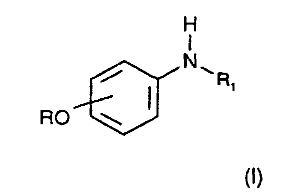 dans laquelle :
R représente un atome d'hydrogène ou un radical -COR₂,
   avec R₂, identique ou différent, représentant un radical choisi parmi un radical alkyle ou alkoxyle, saturé ou insaturé, linéaire, cyclique ou ramifié, en C₁ à C₃₀, éventuellement hydroxylé,
R₁ est choisi parmi un radical de formules (a), (b) ou (c) suivantes :
   (a) -CO-NR₃R₄
   (b) -CO-O-R₅
   (c) -SO₂-R₅
   avec R₃ représentant un atome d'hydrogène ou un radical alkyle en C₁ à C₆, linéaire ou ramifié, saturé ou insaturé, éventuellement hydroxylé,
   avec R₄ représentant un atome d'hydrogène ou le radical R₅ défini ci-dessous,
   avec R₅ représentant un radical choisi parmi un radical alkyle, saturé ou insaturé, linéaire, cyclique ou ramifié, en C₁ à C₃₀, éventuellement hydroxylé. On préfère plus particulièrement parmi ces dérivés d'aminophénol à fonction carbamate, urée ou sulfonamide de formule (I), on préfère le N-éthyloxycarbonyl-4-amino-phénol ; le N-éthyloxycarbonyl-O-éthyloxycarbonyl-4-amino-phénol ; N-cholestéryloxycarbonyl-4-amino-phénol et le N-éthylaminocarbonyl-4-aminophénol.

L'invention a également pour objet l'utilisation d'une telle composition dans le but d'obtenir un blanchiment de la peau à la fois à court et à long terme.

De préférence, une telle composition comprend en outre au moins un agent susceptible de filtrer les UV et/ou au moins un agent desquamant.

L'invention a également pour objet une composition, plus particulièrement cosmétique, comprenant au moins un composé de la famille des azurants optiques et au moins un agent susceptible de filtrer les UV et/ou au moins un agent desquamant.

Parmi les agents susceptibles de filtrer les UV, on peut citer les filtres organiques hydrophiles ou lipophiles et les filtres minéraux.

Les filtres hydrophiles peuvent être notamment choisis parmi les dérivés de la benzophénone, les dérivés de l'acide p-aminobenzoïque, les dérivés du camphre ou encore parmi les dérivés de benzimidazole.

Les filtres hydrophiles peuvent être présents dans la composition finale selon l'invention à une teneur pouvant varier de 0,1 à 20 %, de préférence de 0,2 à 10 %, en poids, par rapport au poids total de la composition.

Les filtres lipophiles convenant particulièrement bien à la présente invention peuvent être choisis parmi les dérivés du dibenzoyméthane, les dérivés de benzimidazole, les dérivés cinnamiques, les dérivés salicyliques, les dérivés du camphre, les dérivés de triazine, les dérivés de la benzophénone, les dérivés de β,β-diphénylacrylate, les dérivés de l'acide p-aminobenzoïque, les polymères filtres et les silicones filtres décrits dans les demandes WO-93/04665 et WO-94/06404. D'autres exemples de filtres organiques sont donnés dans la demande de brevet EP-A 0 487 404.

Le ou les filtres lipophiles peuvent être présents dans la composition finale selon l'invention à une teneur pouvant varier de 0,5 à 30 %, de préférence de 0,5 à 20 %, en poids, par rapport au poids total de la composition.

Les filtres minéraux peuvent être des pigments ou bien encore des nanopigments (taille moyenne des particules primaires : généralement entre 5 nm et 100 nm, de préférence entre 10 et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs bien connus en soi agissant par blocage physique (réflection et/ou diffusion) du rayonnement UV. Des agents d'enrobage classiques sont par ailleurs l'alumine et/ou le stéarate d'aluminium, les silicones. De tels nanopigments d'oxydes métalliques, enrobés ou non enrobés, sont en particulier décrits dans les demande de brevets EP-A- 0 518 772 et EP-A- 0 518 773.

Les filtres minéraux peuvent être présents dans la composition finale selon l'invention à une teneur pouvant varier de 0,1 à 20 %, de préférence de 0,2 à 10 %, en poids, par rapport au poids total de la composition.

Parmi les agents desquamants, on peut citer les hydroxyacides, plus particulièrement les α-, les β-hydroxy acides, l'acide salicylique ou un de ses dérivés, éventuellement salifié, les rétinoïdes naturels ou synthétiques, tels que le rétinol, les esters, l'acide rétinoïque ou le rétinal. Comme dérivés de l'acide salicylique, on peut citer notamment ceux décrits dans les documents FR-A-2581542 et EP-A-378936, et notamment les acides n-octanoyl-5-salicylique, n-décanoyl-5-salicylique, n-dodécanoyl-5-salicylique, n-octyl-5-salicylique, n-heptyloxy-5-salicylique, n-heptyloxy-4-salicylique, ainsi que leurs sels d'ammonium quatemaire tels que les sels de diméthylhydroxypropylammonium. On peut aussi utiliser les dérivés d'acide salicylique décrits dans le document EP-A-570230.

L'invention a en outre pour objet une composition, plus particulièrement cosmétique, comprenant au moins un azurant optique et au moins un agent hydratant. Une telle composition est particulièrement efficace dans la prévention et /ou le traitement des cernes.

Parmi les agents hydratants susceptibles d'être utilisés dans ces compositions, on peut citer en particulier l'urée ou ses dérivés, les polyols, comme par exemple la glycérine, le sorbitol, le propylène glycol.

L'invention a également pour objet une composition, de préférence cosmétique, comprenant au moins un azurant optique et au moins un agent anti-rides. Une telle composition est, elle, aussi particulièrement appropriée à la prévention et/ou au traitement des cernes.

Parmi les agents anti-rides susceptibles d'être utilisés dans ces compositions, on peut citer en particulier : les hydroxyacides, plus particulièrement les α-, les β-hydroxy acides, l'acide ascorbique ou ses dérivés, l'acide salicylique ou un de ses dérivés, éventuellement salifié, les rétinoïdes naturels ou synthétiques, tels que le rétinol, les esters, l'acide rétinoïque ou le rétinal. . Comme dérivés de l'acide salicylique, on peut citer notamment ceux décrits dans les documents FR-A-2581542 et EP-A-378936, et notamment les acides n-octanoyl-5-salicylique, n-décanoyl-5-salicylique, n-dodécanoyl-5-salicylique, n-octyl-5-salicylique, n-heptyloxy-5-salicylique, n-heptyloxy-4-salicylique, ainsi que leurs sels d'ammonium quatemaire tels que les sels de diméthylhydroxypropylammonium. On peut aussi utiliser les dérivés d'acide salicylique décrits dans le document EP-A-570230.

L'invention a enfin pour objet un procédé de blanchiment immédiat de la peau, ce procédé consistant à appliquer sur la peau une composition cosmétique comprenant au moins un azurant optique.

Les compositions contenant les azurants optiques utilisés selon l'invention peuvent présenter toutes les formes galéniques normalement utilisées pour une application topique, par exemple sous forme de solutions, de gels, de dispersions du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle, semi-solide ou solide du type crème ou gel, ou encore de microémulsions, de microcapsules, de microparticules ou de dispersions vésiculaires de type ionique et/ou non ionique. Ces compositions sont préparées selon les méthodes usuelles.

De façon connue, les compositions, plus particulièrement cosmétiques, de l'invention peuvent contenir des adjuvants habituels dans le domaine cosmétique, tels que les émulsionnants, les gélifiants hydrophiles ou lipophiles, les actifs hydrophiles ou lipophiles, les conservateurs, les antioxydants, les parfums, les charges, les matières colorantes. Les quantités de ces différents adjuvants sont celles classiquement utilisées dans les domaines cosmétique, et par exemple de 0,01 à 20 % du poids total de la composition. Ces adjuvants, selon leur nature, peuvent être introduits dans la phase grasse, dans la phase aqueuse et/ou dans les vésicules lipidiques.

Comme émulsionnants, on peut utiliser des émulsionnants eau-dans-huile (E/H) ou huile-dans-eau (H/E) selon l'émulsion finale souhaitée.

Comme émulsionnants, on peut citer par exemple le mélange de stéarate de glycéryle/stéarate de PEG-100 (Arlacel 165 vendu par la société ICI), le stéarate de PEG-20 (Myrj 49 vendu par la société ICI°, le stéarate de PEG-40 (Myrj 52 vendu par la société ICI) et le tristéarate de sorbitan (Span 65 vendu par la société ICI).

Le taux d'émulsionnant peut aller de 0,1 à 15 % en poids, et de préférence de 0,5 à 5 % en poids, par rapport au poids total de la composition.

On peut ajouter à la composition selon l'invention des coémulsionnants, par exemple en une quantité allant de 0,05 % à 10 % en poids par rapport au poids total de la composition. Comme émulsionnant, on peut citer le stéarate de glycérol.

Dans les dispersions de vésicules lipidiques, l'émulsionnant peut être constitué par les vésicules elles-mêmes, de lipides ioniques et/ou non ioniques.

Comme huiles utilisables dans l'invention, on peut citer les huiles minérales, les huiles végétales (huile de germe de maïs), les huiles de synthèse (isohexadécane), les huiles siliconées (cyclométhicone) et les huiles fluorées.On peut utiliser aussi des alcools gras (alcool stéarylique, alcool cétylique), des acides gras (acide stéarique) et des cires.

Comme actifs hydrophiles, on peut utiliser par exemple les protéines ou les hydrolysats de protéine, les acides aminés, l'urée, l'allantoÏne, les sucres et leurs dérivés, l'acide glycyrrhétinique.

Comme actifs lipophiles, on peut utiliser le tocophérol (vitamine E) et ses dérivés, les acides gras essentiels, les céramides, les huiles essentielles.

Ces compositions constituent notamment des crèmes de traitement ou de soin pour le visage, pour les mains ou pour le corps, des laits corporels de protection ou de soin, des lotions, gels ou mousses pour le soin ou le traitement de la peau.

Les exemples suivants illustrent l'invention. Dans ces exemples, les proportions indiquées sont des pourcentages en poids.

### Exemple 1 : crème fluide H/E

- Palmitate d'éthyl-2 hexyle 8 %
- Huile de vaseline 8 %
- Mono-di-isostéarate de glycéryle 2 %
- Mélange de mono-di-stéarate de glycéryle, acide stéarique, glycérine (40/50/5/5) 2 %
- 4,4'-bis[(4,6-dianmilino-1,3,5-triazin-2-yl)amino] stilbène-2,2'-disulfonate de sodium* 1 %
- Triéthanolamine 0,9 %
- Propylène glycol 2 %
- Acide stéarique 2 %
- Stéarate de magnésium 2 %
- Conservateurs qs
- Eau qsp 100
* TINOPAL SOP ® commercialisé par la société CIBA GEIGY

### Exemple 2 : Crème fluide H/E

On prépare une crème avec les mêmes constituants qu'à l'exemple 1, à l'exception du dérivé de stilbène qui est remplacé par 1 % de 2,5-thiophène di-yl bis(5-ter-butyl-1,3-benzoxazole) commercialisé par la société CIBA GEIGY sous le nom de marque UVITEX OB ®.

Ces deux crèmes ont été testées sur des femmes asiatiques et ont été très appréciées pour leur effet blanchissant immédiat, leur transparence. Ces deux crèmes confèrent à la peau un aspect de porcelaine.

### Exemple 3 : crème contour des yeux anti-cernes

- Hyaburonate de sodium 0,1 %
- 4,4'-bis[4,6-dianilino-1,3,5-triazin-2-yl) amino] stilbène-2,2'-disulfonate de sodium* 7 %
- Acide acrylamidométhyl propane sulfonique neutralisé partiellement à l'ammoniaque réticulé 1,5 %
- Glycérine 5 %
- Alcool éthylique 5 %
- Di-oléate de méthyl glucose oxyéthyléné (120 OE) 0,5 %
- Mono-laurate de sorbitane oxyéthyléné (20 OE) 0,5 %
- Conservateurs qs
- Eau qsp 100
* TINOPAL SOP commercialisé par la société CIBA GEIGY

### Exemple 4 : crème contour des yeux anti-cernes

On a reproduit l'exemple 3 avec seulement 1 % de TINOPAL S0P.

Les deux compositions anti-cemes des exemples 3 et 4 ont été testées sur des femmes à peau caucasienne. Toutes deux produisent un effet anti-ceme immédiat, naturel et discret : coloration unifiée de la peau du visage, aspect des traits détendu, aspect flétri du contour des yeux atténué.

## Revendications

1. Utilisation cosmétique d'au moins un composé appartenant à la famille des azurants optiques choisis parmi les composés qui absorbent dans l'UVA entre 300 et 390 nm et réémettent entre 400 et 525 nm, dans une composition comme agent de blanchiment de la peau.

2. Utilisation selon la revendication 1, caractérisée en ce l'azurant optique est un agent de blanchiment immédiat de la peau.

3. Utilisation cosmétique d'au moins un composé appartenant à la famille des azurants optiques choisis parmi les composés qui absorbent dans l'UVA entre 300 et 390 nm et réémettent entre 400 et 525 nm, dans une composition comme agent destiné à conférer au teint au moins une qualité choisie parmi : uniformité, homogénéité, transparence, aspect de porcelaine.

4. Utilisation selon l'une quelconque des revendications 1 à 3, **caractérisée par le fait qu'**elle est destinée au soin des peaux asiatiques.

5. Utilisation cosmétique d'au moins un composé appartenant à la famille des azurants optiques choisis parmi les composés qui absorbent dans l'UVA entre 300 et 390 nm et réémettent entre 400 et 525 nm, dans une composition comme agent anti-cernes.

6. Utilisation selon la revendication 5, **caractérisée par le fait qu'**elle est destinée au soin des peaux caucasiennes.

7. Utilisation selon l'une quelconque des revendications 1 à 6, **caractérisée par le fait que** l'azurant optique est choisi parmi les dérivés du stilbène, les dérivés coumariniques, les dérivés d'oxazole, les dérivés de benzoxazole, les dérivés d'imidazole.

8. Utilisation selon la revendication 7, **caractérisée par le fait que** l'azurant optique est choisi parmi le 4,4'-bis[(4,6-dianilino-1,3,5-triazin-2-yl)amino] stilbène-2,2'-disulfonate de sodium et le 2,5-thiophénedi-yl bis (5-ter-butyl-1,3 benzoxazole).

9. Utilisation selon l'un quelconque des revendications précédentes **caractérisée par le fait que** l'azurant optique est présent dans la composition en quantité allant de 0,1 à 15 %, préférentiellement de 0,5 à 10 % en poids par rapport au poids total de la composition.

10. Composition, **caractérisée par le fait qu'**elle comprend au moins un composé appartenant à la famille des azurants optiques et au moins un second agent de blanchiment de la peau n'appartenant pas à cette famille.

11. Composition selon la revendication 10, **caractérisée par le fait que** le second agent de blanchiment de la peau est choisi parmi ; l'hydroquinone et ses dérivés, l'acide kojique et ses dérivés, l'acide lactique et ses sels, l'acide ascorbique et ses dérivés l'acide azélaïque, l'arbutine et ses dérivés, l'acide L-2-oxothiazolidine 4-carboxylique, le paracétamol et des dérivés d'aminophénol à fonction carbamate, urée ou sulfonamide de formule (I). 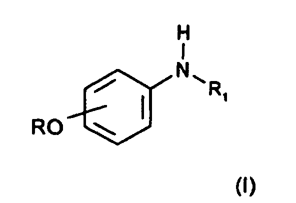 dans laquelle :
R représente un atome d'hydrogène ou un radical -COR₂,
avec R₂, identique ou différent, représentant un radical choisi parmi un radical alkyle ou alkoxyle, saturé ou insaturé, linéaire, cyclique ou ramifié, en C₁ à C₃₀, éventuellement hydroxylé,
R₁ est choisi parmi un radical de formules (a), (b) ou (c) suivantes :
(a) -CO-NR₃R₄
(b) -CO-O-R₅
(c) -SO₂-R₅
avec R₃ représentant un atome d'hydrogène ou un radical alkyle en C₁ à C₆, linéaire ou ramifié, saturé ou insaturé, éventuellement hydroxylé,
avec R₄ représentant un atome d'hydrogène ou le radical R₅ défini ci-dessous,
avec R₅ représentant un radical choisi parmi un radical alkyle, saturé ou insaturé, linéaire, cyclique ou ramifié, en C₁ à C₃₀, éventuellement hydroxylé.

12. Composition selon l'une quelconque des revendications 10 et 11, **caractérisée par le fait qu'**elle comprend en outre au moins un agent susceptible de filtrer les UV.

13. Utilisation d'une composition selon l'une quelconque des revendications 10 à 12, dans le but d'obtenir un blanchiment de la peau.

14. Procédé non thérapeutique de blanchiment immédiat de la peau, **caractérisé par le fait que** l'on applique sur la peau une composition comprenant au moins un composé appartenant à la famille des azurants optiques choisis parmi les composés qui absorbent dans l'UVA entre 300 et 390 nm et réémettent entre 400 et 525 nm.

## Patentansprüche

1. Kosmetische Verwendung mindestens einer Verbindung aus der Gruppe der optischen Aufheller, die unter den Verbindungen ausgewählt ist, die im UV-A-Bereich bei 300 bis 390 nm absorbieren und im Bereich von 400 bis 525 nm emittieren, in einer Zusammensetzung als Wirkstoffe zum Aufhellen der Haut.

2. Verwendung nach Anspruch 1, **dadurch gekennzeichnet, dass** der optische Aufheller ein Wirkstoff zur unmittelbaren Aufhellung der Haut ist.

3. Kosmetische Verwendung mindestens einer Verbindung aus der Gruppe der optischen Aufheller, die unter in Verbindungen, die im UV-A-Bereich bei 300 bis 390 nm absorbieren und im Bereich von 400 bis 525 nm emittieren, in einer Zusammensetzung als Wirkstoff, der dazu vorgesehen ist, dem Teint mindestens eine der folgenden Eigenschaften zu verleihen: Gleichförmigkeit, Homogenität, Transparenz und porzellanartiges Aussehen.

4. Verwendung nach einem der Ansprüche 1 bis 3, **dadurch gekennzeichnet, dass** sie zur Pflege von asiatischen Hauttypen vorgesehen ist.

5. Kosmetische Verwendung mindestens einer Verbindung aus der Gruppe der optischen Aufheller, die unter den Verbindungen ausgewählt sind, die im UV-A-Bereich bei 300 bis 390 nm absorbieren und im Bereich von 400 bis 525 nm emittieren, in einer Zusammensetzung als Wirkstoff gegen Augenringe.

6. Verbindung nach Anspruch 5, **dadurch gekennzeichnet, dass** sie zur Pflege von kaukasischen Hauttypen vorgesehen ist.

7. Verwendung nach einem der Ansprüche 1 bis 6, **dadurch gekennzeichnet, dass** der optische Aufheller unter den Stilbenderivaten, Cumarinderivaten, Oxazolderivaten, Benzoxazolderivaten und Imidazolderivaten ausgewählt ist.

8. Verwendung nach Anspruch 7, **dadurch gekennzeichnet, dass** der optische Aufheller unter Natrium-4,4'-bis[(4,6-dianilino-1,3,5-triazin-2-yl)amino]stilben-2,2'-disulfonat und 2,5-Thiophendi-yl-bis(5-*t*-butyl-1,3-benzoxazol) ausgewählt ist.

9. Verwendung nach einem der vorhergehenden Ansprüche, **dadurch gekennzeichnet, dass** der optische Aufheller in der Zusammensetzung in einer Menge von 0,1 bis 15 % und vorzugsweise 0,5 bis 10 Gew.-%, bezogen auf das Gesamtgewicht der Zusammensetzung, enthalten ist.

10. Zusammensetzung, **dadurch gekennzeichnet, dass** sie mindestens eine Verbindung aus der Gruppe der optischen Aufheller und mindestens einen zweiten Wirkstoff zur Aufhellung der Haut, der nicht zu dieser Gruppe gehört, enthält.

11. Zusammensetzung nach Anspruch 10, **dadurch gekennzeichnet, dass** der zweite Wirkstoff zum Aufhellen der Haut ausgewählt ist unter: Hydrochinon und seinen Derivaten, Kojisäure und ihren Derivaten, Milchsäure und ihren Salzen, Ascorbinsäure und ihren Derivaten, Azelainsäure, Arbutin und seinen Derivaten, L-2-Oxothiazolidin-4-carbonsäure, Paracetamol und Aminophenolderivaten mit Carbamat-, Harnstoff- oder Sulfonamidfunktion der Formel (I): 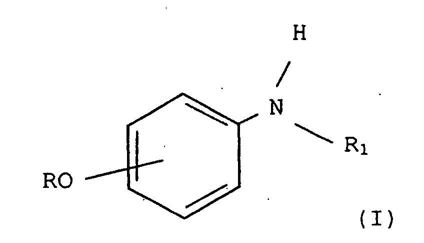 worin bedeuten:
R ein Wasserstoffatom oder eine Gruppe -COR₂, wobei die Gruppen R₂, die identisch oder voneinander verschieden sind, eine Gruppe bedeuten, die unter den gesättigten oder ungesättigten, geradkettigen, cyclischen oder verzweigten, gegebenenfalls hydroxylierten Alkyl- oder Alkoxygruppen mit 1 bis 30 Kohlenstoffatomen ausgewählt sind,
R₁ eine Gruppe, die unter den folgenden Gruppen der Formeln (a), (b) oder (c) ausgewählt ist:
(a) -CO-NR₃R₄,
(b) -CO-O-R₅,
(c) -SO₂R₅,
wobei
R₃ ein Wasserstoffatom oder eine geradkettige oder verzweigte, gesättigte oder ungesättigte, gegebenenfalls hydroxylierte C₁₋₆-Alkylgruppe bedeutet,
R₄ ein Wasserstoffatom oder die nachstehend definierte Gruppe R₅ bedeutet, und
R₅ eine Gruppe bedeutet, die unter den gesättigten oder ungesättigten, geradkettigen, cyclischen oder verzweigten C₁₋₃₀-Alkylgruppen, die gegebenenfalls hydroxyliert sind, ausgewählt ist.

12. Zusammensetzung nach einem der Ansprüche 10 und 11, **dadurch gekennzeichnet, dass** sie ferner mindestens einen Wirkstoff enthält, der die UV-Strahlung filtern kann.

13. Verwendung einer Zusammensetzung nach einem der Ansprüche 10 bis 12 zur Aufhellung der Haut.

14. Nichttherapeutisches Verfahren zur unmittelbaren Aufhellung der Haut, das darin besteht, auf die Haut eine Zusammensetzung aufzutragen, die mindestens eine Verbindung aus der Gruppe der optischen Aufheller enthält, die unter den Verbindungen ausgewählt sind, die im UV-A-Bereich im Bereich von 300 bis 390 nm absorbieren und im Bereich von 400 bis 525 nm reemittieren.

## Claims

1. Cosmetic use of at least one compound belonging to the optical brightener family, the said optical brightener being chosen from compounds which absorb in the UVA range between 300 and 390 nm and re-emit between 400 and 525 nm in a composition as a skin bleaching agent.

2. Use according to Claim 1, **characterized in that** the optical brightener is an agent for the immediate bleaching of the skin.

3. Cosmetic use of at least one compound belonging to the optical brightener family, the said optical brightener being chosen from compounds which absorb in the UVA range between 300 and 390 nm and re-emit between 400 and 525 nm in a composition as an agent intended to give the complexion at least one quality chosen from: uniformity, homogeneity, clarity, alabaster-smooth appearance.

4. Use according to any one of Claims 1 to 3, **characterized in that** it is intended for the care of Asian skin.

5. Cosmetic use of at least one compound belonging to the optical brightener family, the said optical brightener being chosen from compounds which absorb in the UVA range between 300 and 390 nm and re-emit between 400 and 525 nm in a composition as a concealer agent.

6. Use according to Claim 5, **characterized in that** it is intended for the care of Caucasian skin.

7. Use according to any one of Claims 1 to 6, **characterized in that** the optical brightener is chosen from stilbene derivatives, coumarin derivatives, oxazole derivatives, benzoxazole derivatives and imidazole derivatives.

8. Use according to Claim 7, **characterized in that** the optical brightener is chosen from sodium 4,4'-bis [(4,6-dianilino-1,3,5-triazin-2-yl)amino]stilbene-2,2'-disulphonate and 2,5-thiophenediylbis(5-tertbutyl-1,3-benzoxazole).

9. Use according to any one of the preceding claims, **characterized in that** the optical brightener is present in the composition in an amount ranging from 0.1 to 15%, preferably from 0.5 to 10%, by weight relative to the total weight of the composition.

10. Composition, **characterized in that** it comprises at least one compound belonging to the optical brightener family and at least a second skin bleaching agent not belonging to this family.

11. Composition according to Claim 10, **characterized in that** the second skin bleaching agent is chosen from: hydroquinone and its derivatives, kojic acid and its derivatives, lactic acid and its salts, ascorbic acid and its derivatives, azelaic acid, arbutin and its derivatives, L-2-oxothiazolidine-4-carboxylic acid, paracetamol and aminophenol derivatives containing a carbamate, urea or sulphonamide function of formula (I) 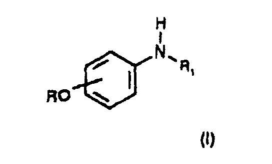 in which:
R represents a hydrogen atom or a radical -COR₂,
with R₂, which may be identical or different, representing a radical chosen from a saturated or unsaturated, linear, cyclic or branched, optionally hydroxylated, C₁ to C₃₀ alkyl or alkoxy radical,
R₁ is chosen from a radical of formula (a), (b) or (c) below:
(a) -CO-NR₃R₄
(b) -CO-O-R₅
(C) -SO₂-R₅
with R₃ representing a hydrogen atom or a linear or branched, saturated or unsaturated, optionally hydroxylated C₁ to C₆ alkyl radical,
with R₄ representing a hydrogen atom or the radical R₅ defined below,
with R₅ representing a radical chosen from a saturated or unsaturated, linear, cyclic or branched, optionally hydroxylated, C₁ to C₃₀ alkyl radical.

12. Composition according to either of Claims 10 and 11, **characterized in that** it also comprises at least one agent capable of screening out UV rays.

13. Use of a composition according to any one of Claims 10 to 12, for the purpose of bleaching the skin.

14. Non-therapeutic process for the immediate bleaching of the skin, **characterized in that** a composition comprising at least one compound belonging to the optical brightener family, the said optical brighteners being chosen from compounds which absorb in the UVA range between 300 and 390 nm and re-emit between 400 and 525 nm, is applied to the skin.
